(19) Europäisches Patentamt
European Patent Office
Office européen des brevets

(11) **EP 3 501 504 A1**

(12) **EUROPEAN PATENT APPLICATION**

(43) Date of publication:
**26.06.2019 Bulletin 2019/26**

(51) Int Cl.:
***A61K 9/20*** *(2006.01)*      ***A61K 9/28*** *(2006.01)*

(21) Application number: **17460079.1**

(22) Date of filing: **22.12.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Pharmacia Polonica sp. z o.o.**
**00-725 Warszawa (PL)**

(72) Inventors:
• **Kieronska, Hanna Maria**
**62-051 Wiry (PL)**

• **Berdzinska, Katarzyna**
**62-095 Murowana Goslina (PL)**
• **Bernkop-Schnürch, Andreas**
**6080 Igls (AT)**
• **Lagiewka, Wojciech**
**00-714 Warszawa (PL)**
• **Zwolinska-Obarska, Anna**
**02-904 Warszawa (PL)**
• **Rapacz, Joanna**
**05-400 Otwock (PL)**
• **Nejman, Michal**
**01-876 Warszawa (PL)**

(54) **MODIFIED RELEASE TABLET OF PALIPERIDONE AND A PROCESS FOR THE PREPARATION THEREOF**

(57) The present invention relates to a modified release tablet of paliperidone, characterised in that the tablet core comprises: 0.1% to 6% of paliperidone, 30% to 40% of a mixture of lightly cross-linked carbomer and highly cross-linked carbomer, wherein the ratio of lightly cross-linked carbomer to highly cross-linked carbomer is from 1:1 to 10:1, 50 to 65% of filler selected from lactose or dibasic calcium phosphate, wherein the tablet core further comprises mannitol, talc, silicon dioxide and magnesium stearate.

**EP 3 501 504 A1**

## Description

### Technical field

The invention relates to a modified release tablet of paliperidone for oral use in humans. Furthermore, the invention relates to a process for the preparation of a modified release tablet comprising paliperidone.

### Background of the invention

Paliperidone is a hydroxyl derivative and active metabolite of risperidone. It is used as atypical antipsychotic drug for the treatment of schizophrenia, bipolar disorder and schizoaffective disorder. Chemically paliperidone is a benzisoxasole derivative. Paliperidone is also known as 3-(2-(4-(6-fluoro-3-(1,2-benzisoxazolyl))-1-piperidinyl)ethyl)-6,7,8,9-tetrahydro-9-hydroxy-2-methyl-4H-pyrido(1,2-a)-pyrimidin-4-one and has the CAS number 144598-75-4. A method of paliperidone manufacturing was described, for example, in US 5,158,952. Paliperidone can be also obtained by other methods e.g., as described in US2009/0048272. Paliperidone is practically insoluble in water. Its solubility in water is 0.003 g/100 ml. Solubility of paliperidone in aqueous solution is pH dependent, rising to 2.3 g / 100 ml in 0.1 N HCl. Pharmaceutical formulation of paliperidone is commercially available as extended-release tablet under the tradename Invega. Formulation of the Invega medicinal product is based on OROS technology, wherein the active substance is released in an osmotic manner. Invega medicinal product has the form of trilayer tablet, coated with semipermeable membrane with laser drilled orifices of specific diameter. The active substance is released as a result of water penetration through the membrane and expansion of the osmotic layer, which pushes initially the first portion and further the second portion of the product through the drilled orifices. In consequence, the active substance is released from the tablet in pH independent manner with almost zero kinetics. Substantially full release of the active substance is achieved after around 18-24h. The methods of manufacturing of osmotic dosage forms were described e.g. in WO1998/06380, US2005/0025831 and US2009/0202631.

Due to complex process, the manufacturing of OROS tablets is expensive, which may translate to high price of the drug product and its limited availability for the patients. One of the disadvantages of taking OROS tablets is the fact, that they are not digested and are excreted in an unchanged form, which may cause discomfort for some of the patients.

Other extended-release dosage forms of paliperidone are known, e.g., in the form of pellets or matrix tablets. EP2161019 A1 discloses pharmaceutical compositions of paliperidone in a form of pellets, which are coated with delayed release coating, wherein single pellet has a diameter not larger than 2 mm. WO2010/009900 discloses a matrix tablet of paliperidone, wherein the matrix of the tablet comprises a combination of matrix forming agents selected from the group either consisting of a combination of neutral swelling polymer with an ionic swelling polymer or a combination of a non-ionic hydrophilic polymer with an anionic hydrophilic polymer.

There is still a need for development of economically viable pharmaceutical formulation of paliperidone, which provides extended release of the active substance and is convenient for the patient.

### Summary of the invention

The invention relates to pharmaceutical formulation of paliperidone in a form of modified release matrix tablet, which is prepared by direct compression and wherein the tablet core comprises at least two types of carbomers with different degree of cross-linking and in specific ratio.

Furthermore, the invention provides modified release matrix tablet of paliperidone, which releases the active substance substantially with zero order kinetics.

The modified release tablet of paliperidone according to the invention may be further film-coated or coated, wherein the coating can modify the initial release profile of the tablet core.

The invention provides modified release tablet comprising pharmaceutically effective amount of paliperidone, having a release profile of the active substance, which is similar to a release profile of the commercial product Invega.

The process for the preparation of the tablet according to the invention is inexpensive and may be performed with the use of basic equipment in repeatable manner. Furthermore, during the preparation of the tablet according to the present invention there is no need to use organic solvents or wet granulation steps, which makes the process eco-friendly.

The authors of the present invention unexpectedly found that similar paliperidone release kinetics to the commercially available tablet Invega is achieved by matrix tablet comprising specific combination of carbomers in the tablet core. The initial paliperidone release profile from such tablet core is optionally modified by the use of functional coating (film-coating) of the tablet core, e.g. with the use of film-coat based on ethylcellulose.

**Brief description of the drawings**

Figure 1 illustrates the release profile of paliperidone from the tablets according to example 1.
Figure 2 illustrates the release profile of paliperidone from the tablets according to example 2.

**Detailed description of the invention**

Definitions

The term "paliperidone" or "active substance" as used herein includes paliperidone free base, hydrates, solvates or polymorphs thereof. Polymorphs of paliperidone include crystalline form I and crystalline form II.

The term "carbomer" or "carbomers" as used herein relates to high molecular mass polymers of acrylic acid cross-linked with polyalkenyl ethers of sugars or polyalcohols. Carbomers suitable for the present invention are hydrophilic lightly and highly cross-linked polyacrylic acid homopolymers, which are commercially available under the tradename Carbopol. These anionic polymers are synthesized from acrylic acid cross-linked with allyl sucrose or allyl pentaerythritol in either ethyl acetate or a cosolvent ethyl acetate/cyclohexane mixture. Being ionically bound to such polymers the cationic substance paliperidone is slowly released. Preferred carbomers are lightly cross-linked such as Carbopol 971P NF or Carbopol 71G NF and highly cross-linked such as Carbopol 974P NF manufactured by Lubrizol company. Further specifications of Carbopol polymers can be found at Lubrizol web site www.lubrizol.com. Carbomers suitable for the present invention comply with Ph. Eur. monograph 1299 "Carbomers".

The term "lightly cross-linked carbomer" as used herein relates to carbomer which has viscosity of 4000 to 11000 cP when measured as 0.5% aqueous solution neutralized to pH 7.3 - 7.8 with Brookfield viscometer at 20 rpm at 25°C.

The term "highly cross-linked carbomer" as used herein relates to carbomer, which has viscosity of 29400 to 39400 cP when measured as 0.5% aqueous solution neutralized to pH 7.3 - 7.8 with Brookfield viscometer at 20 rpm at 25°C.

The word "substantially" as used herein represents general rule or trend and includes some degree of error or variability associated with measurement of the particular quantity.

The term "substantially zero order release kinetics" as used herein relates to release of the substance at a substantially constant rate. For dissolution studies, normally, the permitted range in release at any given time point should not exceed a total numerical difference of $\pm10\%$ of the labelled content of active substance (i.e. a total variability of 20%: a requirement of $50\pm10\%$ thus means an acceptable range from 40-60%),

Definitions and conventions relating to "modified release tablet" or other modified release dosage forms follow general pharmaceutical guidelines, such as, in particular, "Guideline on quality of oral modified release products" EMA/CHMP/QWP/428693/2013.

Amounts provided in percent refer to weight/weight percent (% w/w) unless otherwise indicated herein or otherwise clearly contradicted by context.

In the first aspect, the present invention provides modified release tablet of paliperidone, wherein the tablet core comprises 0.1% to 6% of paliperidone, 30% to 40% of a mixture of lightly cross-linked carbomer and highly cross-linked carbomer, wherein the ratio of lightly cross-linked carbomer to highly cross-linked carbomer is from 1:1 to 10:1, 50 to 65% of filler selected from lactose or dibasic calcium phosphate, wherein the tablet core further comprises mannitol, talc, silicon dioxide and magnesium stearate.

In a preferred embodiment, the tablet core comprises pharmaceutically effective amount of paliperidone.

In a preferred embodiment, paliperidone is in a form of free base, crystalline form.

In some embodiments, the tablet core comprises paliperidone in amount of at least 0.1%, at least 1.5%, at least 3% at least 4.5%, or 6% of the weight of the tablet core.

In one embodiment, the tablet core comprises paliperidone in amount of 3 mg.

In another embodiment, the tablet core comprises paliperidone in amount of 6 mg.

In another embodiment, the tablet core comprises paliperidone in amount of 9 mg.

In another embodiment, the tablet core comprises paliperidone in amount of 12 mg.

In a preferred embodiment, the tablet core of the modified release tablet of paliperidone according to the invention comprises lightly cross-linked carbomer, which has viscosity of 4000 to 11000 cP and highly cross-linked carbomer, which has viscosity of 29400 to 39400 cP when measured as 0.5% aqueous solution neutralized to pH 7.3 - 7.8 with Brookfield viscometer at 20 rpm at 25°C.

Preferably the ratio of lightly cross-linked carbomer to highly cross-linked carbomer in the tablet core is from 1:1 to 10:1. More preferably the ratio of lightly cross-linked carbomer to highly cross-linked carbomer in the tablet core is 1:1. Most preferably the ratio of lightly cross-linked carbomer to highly cross-linked carbomer in the tablet core is 9.3:1.

In one embodiment powdered carbomers are used in the tablet core. Powdered carbomers are characterised by primary particles of average size from 0.2 $\mu$m to 6 $\mu$m. Preferably powdered carbomers in amount of at least 5%, at least 6%, at least 30% or in amount of 40% of the weight of the tablet core are used in the tablet core. Most preferably powdered carbomers in amount of at least 6% of the weight of the tablet core are used in the tablet core.

In another embodiment granulated carbomers are used in the tablet core. Granulated carbomers are characterised by particles, where at least 95% of particles pass through 40 mesh (425 $\mu$m) and not more than 10% of particles pass through 100 mesh (150 $\mu$m). Preferably granulated carbomers in amount of at least 25%, at least 31% or in amount of 40% of the weight of the tablet core are used in the tablet core. Most preferably granulated carbomers in amount of at least 25% of the weight of the tablet core are used in the tablet core.

In preferred embodiment the combination of powdered and granulated carbomers is used. Preferably powdered carbomers in amount of at least 5% or at least 6% of the weight of the tablet core are used in the tablet core in combination with granulated carbomer in amount of at least 25% of the weight of the tablet core. Most preferably powdered carbomers in amount of 6% of the weight of the tablet core are used in the tablet core in combination with granulated carbomer in amount of 25% of the weight of the tablet core.

In preferred embodiment the tablet core comprises a filler and other excipients which are compatible with paliperidone and with each other. Preferably the tablet according to the invention comprises 50 - 65% of the filler in the tablet core.

In one embodiment, different strengths of the modified release tablet of paliperidone with similar release characteristics are prepared based on the same total amount of paliperidone and filler taken together, e.g., for 3 mg strength, a tablet that contains 3 mg of paliperidone and 100 mg of the filler can be prepared, wherein for 6 mg strength, a tablet that contains 6 mg of paliperidone and 97 mg of the filler can be prepared, so the total amount of paliperidone and filler taken together remains constant (103 mg) in both strengths.

Preferable fillers according to the present invention are lactose or dibasic calcium phosphate dihydrate.

Lactose as a filler suitable for the invention is chosen from any lactose that is commonly used for direct compression and includes anhydrous lactose and lactose monohydrate. Preferably lactose is spray dried lactose monohydrate for direct compression. Preferably lactose is Ph. Eur. compliant, e.g., in the form as it is marketed by DFE Pharma under the trade name Supertab 14SD.

Dibasic calcium phosphate as a filler suitable for the invention is chosen from any dibasic calcium phosphate used for direct compression and includes anhydrous and hydrated forms. Preferably dibasic calcium phosphate dihydrate for direct compression is used. Preferably dibasic calcium phosphate dihydrate is Ph. Eur. compliant, e.g., in the form as it is marketed by JRS Pharma under the trade name Emcompress.

In a preferred embodiment, the tablet core comprises lactose as a filler in amount from 50% to 65% of the weight of the tablet core. More preferably, the tablet core comprises lactose in amount from 50% to 55% or from 55% to 60% or from 60% to 65% of the weight of the tablet core. Most preferably, the tablet core comprises lactose in amount of 59% of the weight of the tablet core.

In preferred embodiment, the tablet core comprises mannitol as excipient. Preferably mannitol is used in amount of 4% of the weight of the tablet core.

In preferred embodiment, the tablet core comprises talc as excipient. Preferably talc is used in amount of 1% of the weight of the tablet core.

In preferred embodiment, the tablet core comprises silicon dioxide as excipient. Preferably silicon dioxide is used in amount of 1% of the weight of the tablet core.

In preferred embodiment, the tablet core comprises magnesium stearate as excipient. Preferably magnesium stearate is used in amount of 1% of the weight of the tablet core.

In preferred embodiment, the tablet core comprises mannitol, talc, silicon dioxide and magnesium stearate as excipients. Such combination of said excipients, including said carbomers in a preferred ratio, yields free flowing blend, with good compressibility and the resulting tablet core releases the active substance with substantially zero order kinetics.

If preferred, other excipients, pigments, flavouring agents or other active substance(s) can be included in the tablet core of modified release tablet of paliperidone according to the invention.

Preferably the tablet core according to the present invention is prepared by direct compression. Preferably nominal weight of the tablet core is 200 mg.

In one embodiment, a process for the preparation of a modified release tablet of paliperidone comprises the steps of:

    a) dry blending of paliperidone with a mixture of lightly cross-linked carbomer and highly cross-linked carbomer, mannitol, talc, silicon dioxide, filler and magnesium stearate,
    b) direct tabletting of the resulting blend,
    c) optionally film-coating of the obtained tablets,

d) optionally curing the obtained film-coated tablets,

wherein tablet core obtained in step b) comprises 0.1% to 6% of paliperidone, 30% to 40% of a mixture of lightly cross-linked carbomer and highly cross-linked carbomer, wherein the ratio of lightly cross-linked carbomer to highly cross-linked carbomer is from 1:1 to 10:1 and wherein the tablet core obtained in step b) comprises 50 to 65% of filler selected from lactose or calcium phosphate dibasic.

In preferred embodiment, a process for the preparation of a modified release tablet of paliperidone comprises the steps of:

a) preparation of intragranular phase by: dry blending of paliperidone with a mixture of lightly cross-linked carbomer and highly cross-linked carbomer, mannitol, talc, silicon dioxide, part of filler and part of magnesium stearate; dry compression of the resulting first blend; milling of the tablets obtained to form intragranular phase,
b) blending of the intragranular phase from step a) with granular lightly cross-linked carbomer, second part of filler and second part of magnesium stearate,
c) direct tabletting of the second blend obtained in step b)
d) optionally film-coating of the tablets obtained in step c)
e) optionally curing the film-coated tablets obtained in step d),

wherein tablet core obtained in step c) comprises 0.1% to 6% of paliperidone, 30% to 40% of a mixture of lightly cross-linked carbomer and highly cross-linked carbomer, wherein the ratio of lightly cross-linked carbomer to highly cross-linked carbomer is from 1:1 to 10:1 and wherein the tablet core obtained in step c) comprises 50% to 65% of filler selected from lactose or calcium phosphate dibasic.

Preferably dry compression of the first blend is performed using tablet press or roller compactor. Most preferably dry compression of the first blend is performed using tablet press. Preferably tablet press is selected form a group of presses commonly used in pharmaceutical industry for direct compression, including single punch tablet press or rotary tablet press.

Preferably direct compression of the second blend is performed using tablet press. Most preferably the same tablet press is used for dry compression of the first blend and direct compression of the second blend.

In one embodiment, the modified release tablet of paliperidone according to the present invention is an uncoated tablet, also referred as a tablet core. Preferably, an uncoated tablet or a tablet core according to the invention releases paliperidone with substantially zero order kinetics. More preferably, an uncoated tablet or a tablet core according to the invention releases from 4% to 5% of the total amount of paliperidone per hour, when measured using paddle dissolution apparatus at pH 6.8 in 500 ml of phosphate buffer at 37°C and 50 rpm.

In broad aspect, the present invention provides a modified release tablet of paliperidone, wherein majority of the paliperidone is released with substantially zero order release kinetics. The majority of paliperidone is to be understood as 80% of the paliperidone dissolved or released (Q=80%).

In a preferred embodiment, the present invention provides a modified release tablet of paliperidone, showing substantially zero order release kinetics, with release rate of 4% to 5% of the total amount of paliperidone per hour.

In another embodiment, the present invention provides a film-coated modified release tablet of paliperidone, showing substantially zero order release kinetics, with release rate of 4% to 5% of the total amount of paliperidone per hour and a lag time from 0 up to 4 hours.

Preferably film-coating of the tablet core according to the invention is performed using water insoluble polymer. More preferably film-coating is performed using ethylcellulose. Most preferably film-coating is performed using ethylcellulose together with a pore former and a plasticiser. The presence of a pore former increases the porosity of an insoluble polymer film and facilitates active substance diffusion. Preferably a pore former is selected from a group of water soluble polymers, such as polyethylene glycol or hydroxypropylmethylcellulose. Most preferably hydroxypropylmethylcellulose is used as a pore former. Plasticizers are non-volatile, high-boiling liquids used to impart flexibility to otherwise hard or brittle polymeric materials. Preferable plasticizers for the invention include dibutyl sebacate (DBS), acetylated monoglycerides, triacetin (GTA or glyceryl triacetate), acetyltriethyl citrate (ATEC) and triethyl citrate (TEC). Most preferably in the present invention triethyl citrate is used as a plasticizer.

In one embodiment, the tablet core of modified release tablet of paliperidone according to the invention is film-coated using 30% ethylcellulose water dispersion (Aquacoat ECD 30 manufactured by FMC Biopolymer) and hydroxypropylmethylcellulose as a pore former and triethyl citrate as a plasticizer. Preferably film-coating is applied up to about 5 to 10% increase of the tablet core weight. Most preferably film-coating is applied up to 5% increase of the tablet core weight.

In yet another embodiment, the film-coated modified release tablet of paliperidone according to the invention is cured directly after film-coating. Curing of the film-coated tablets stabilizes the film-coating layer and insures reproducible release profiles during storage. Preferably curing is performed by heating of the film-coated tablets at 60°C for two hours. Most preferably curing is performed by heating of the film-coated tablets at 60°C for two hours at ambient humidity.

## Examples

### Example 1

Modified release tablets of paliperidone were prepared according to the composition presented in Table 1.

Table 1.

| Ingredients | Composition [%] w/w per tablet core |
| --- | --- |
| Paliperidone | 3 |
| Dibasic calcium phosphate dihydrate | 50 |
| Highly cross-linked carbomer Carbopol 974P NF | 20 |
| Lightly cross-linked carbomer Carbopol 971P NF | 20 |
| Mannitol | 4 |
| Talc | 1 |
| Silicon dioxide (Aerosil 200) | 1 |
| Magnesium stearate | 1 |

Paliperidone, dibasic calcium phosphate dihydrate, powdered highly cross-linked carbomer Carbopol 974P NF, powdered lightly cross-linked carbomer Carbopol 971P NF, mannitol, talc, silicon dioxide Aerosil 200 and magnesium stearate were dry blended. After addition of each excipient the mixture was grinded/blended for at least 2 min. The whole mixture was passed through a 1.5 mm sieve, filled in a plastic beaker and shaken for 30 min at 700 rpm on a shaking board. Thereafter, tablets were pressed and tested for their drug release behaviour.

Dissolution studies were performed each at 37°C and 50 rpm for 24 h using paddle apparatus. After 2, 4, 6, 8, 12, 18 and 24 h samples of 5 ml were withdrawn and the concentration of Paliperidone in withdrawn samples quantified via HPLC. Tablets had a diameter of 13 mm, a weight of 200 mg and a drug concentration of 3%. Total amount of paliperidone in one tablet was 6 mg. As release media 500 ml of modified SGF pH 1.0 [NaCl (0.2% m/m) in 0.0825 M HCl] and 500 ml of phosphate buffer pH 6.8 [phosphate buffer pH 6.8 according to Ph. Eur. 5.17.1] were used.

Similarity factor (f2) is commonly used to establish similarity of two dissolution profiles and is the logarithmic transformation of the sum-squared error of differences between the test $T_t$ and reference products $R_t$ over all time points. It represents closeness of two comparative formulations. An average difference of 10% at all measured time points results in a f2 value of 50. Generally, similarity factor in the range of 50-100 is acceptable according to US FDA and indicate similarity between two dissolution profiles. Similarity factor f2 for prepared tablets of the test product (according to the present example) with the reference product Invega 6 mg, prolonged-release tablets, was calculated based on the following equation:

$$f_2 = 50 + \log \{[1 + (1/n)_{t=1} * n \, (R_t - T_t)^2]^{-0.5} * 100\}$$

where, $R_t$ and $T_t$ are the cumulative percentage dissolved at each of the selected n time points of the reference and test product, respectively.

Similarity factor f2 for the prepared tablets in medium of pH 6.8 was 50.1, which means that dissolution profiles were similar.

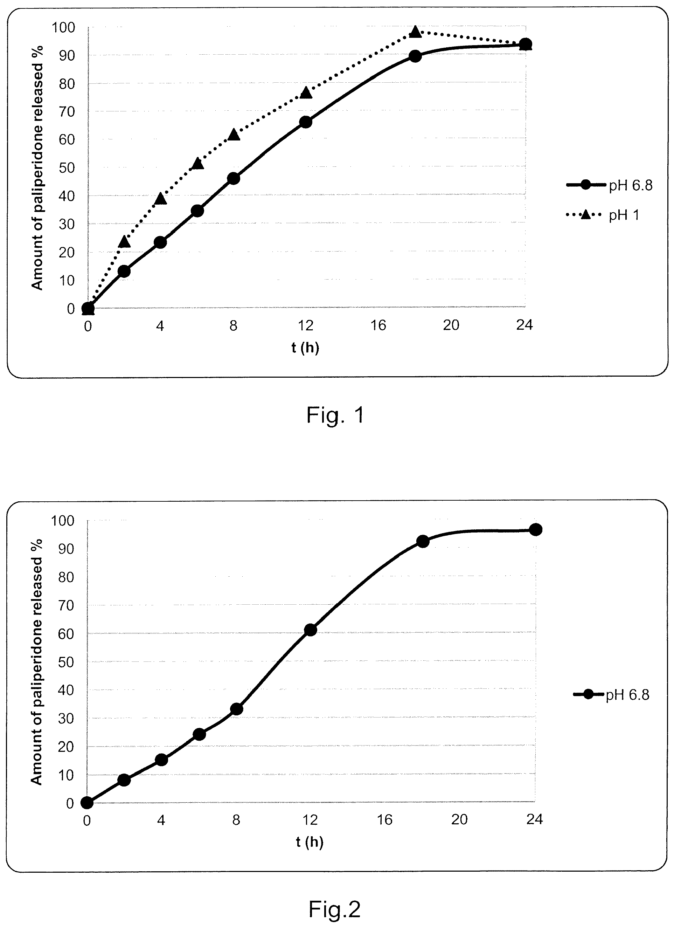

Dissolution profile of the tablets prepared according to example 1, in two media of different pH, are presented in Fig. 1

### Example 2

Modified release tablets of paliperidone were prepared according to composition provided in Table 2.

Table 2.

| Ingredients in intragranular phase | Composition [%] w/w per tablet core |
|---|---|
| Paliperidone | 3.00 |
| Lactose monohydrate (Supertab 14SD) | 49.00 |
| Highly cross-linked carbomer Carbopol 974P NF | 3.00 |
| Lightly cross-linked carbomer Carbopol 971P NF | 3.00 |
| Mannitol | 4.00 |
| Talc | 1.00 |
| Silicon dioxide (Aerosil 200 VV) | 1.00 |
| Magnesium stearate | 0.80 |
| **Ingredients in extragranular phase** | **Composition [%] w/w per tablet core** |
| Intragranular phase | 64.80 |
| Lightly cross-linked carbomer Carbopol 71G NF | 25.00 |
| Lactose monohydrate (Supertab 14SD) | 10.00 |
| Magnesium stearate | 0.20 |

The tablets were prepared according to the following procedure with amounts indicated in Table 2 for each of the phases.

1. Preparation of the Intragranular phase:

Intragranular phase was prepared in nominal batch size 1296 g as follows:
Raw materials were weighed. Around 1/3 part of the lactose, and further paliperidone, lightly cross-linked carbomer Carbopol 971P NF, talc, highly cross-linked carbomer Carbopol 974P NF and mannitol were added to metal pan and preblended manually. The blend was sieved through 0.75 mm sieve to 8L blender. The remaining part of lactose, and full amount of silicon dioxide (Aerosil 200 VV) were added and the mixture was blended for 15 min at 17-18 rpm. Magnesium stearate, sieved through 0.5 mm sieve was then added and the mixture was blended for additional 3 minutes at 17-18 rpm.

2. Tabletting of the resulting blend:

Intragranular phase was compressed using rotary press Korsch XL100 EU-D with 7.5 mm punches. Powdered blend was added in portions, which facilitated powder flow. The resulting tablets had mean weight of 180.8 mg and mean hardness of 53.6 N.

3. Milling of the tablets:

Tablets prepared in the previous step were milled using Quadro Comil U5 conical mill with 062G sieve and rectangular impeller at 2500 - 3500 rpm. Some part of the material was used for analytical testing and finally 1064.6 g of free flowing intragranular phase was obtained. Material was divided into three parts of 350.0 g each

4. Blending with the remaining excipients:

Based on the amount of intragranular phase (350.0 g), the amounts of excipients to add to extragranular phase were recalculated. Granulated lightly cross-linked carbomer Carbopol 71G NF and lactose monohydrate were sieved through 0.75 mm sieve and added to the intragranular phase obtained at step 3. The mixture was blended for 15 min at 17-18 rpm. Magnesium stearate, sieved through 0.5 mm sieve was then added and the mixture was blended for additional 3 minutes at 17-18 rpm.

5. Final tabletting:

Final blend was added in portions and compressed using 7.5 mm punches on rotary press. The mean weight of the tablets was 198 mg and the mean tablet hardness was 63 N.

Similarity factor f2 for prepared tablets of the test product with the reference product Invega 6 mg, prolonged-release tablets was calculated as provided in Example 1.

Similarity factor f2 for the prepared tablets dissolved in 500 ml of the medium of pH 6.8 at 50 rpm and 37°C in paddle apparatus was 51, which means that dissolution profiles were similar.

Dissolution profile of the prepared tablets was provided in Fig. 2.

Example 3

Tablets prepared according to the process described in example 2 were film-coated with the use of the film-coating composition as presented in Table 3

Table 3

| Ingredient | Amount [g] |
|---|---|
| 30% water dispersion of ethylcellulose (Aquacoat ECD) | 100.0 |
| Pore former - Hydroxypropylmethylcellulose (Pharmacoat 606) | 3.0 |
| Plasticizer - triethyl citrate (TEC) | 7.2 |
| Water, purified | 157.8 |

To the flask with weighed amount of Aquacoat ECD, TEC was added in portions with stirring. Stirring was continued for 60 min at 450 rpm. Pharmacoat 606 was dissolved in 100.0 g of purified water and stirred for 45 min at 260 rpm. The resulting solution of Pharmacoat 606 was added to the mixture of Aquacoat ECD and TEC following with addition of the remaining amount of water. The resulting mixture was stirred for 15 min at 450 rpm and filtered through 0.5 mm sieve.

The tablets were film-coated with the prepared film-coating solution in O'Hara Labcoat M coating machine using 1.2 mm nozzle.

After 40 min. 5% increase in mass was recorder, after 56 min. 7% increase in mass was recorded and after 88 min. 12% increase in mass was recorded and the film-coating process was stopped at this stage. The surface of the tablets was evenly coated.

Film-coated tablets with 5% increase in mass were cured directly after coating by heating to 60°C for 2 h in ambient relative humidity conditions. The film-coated tablets were allowed to cool to room temperature and were packed in polyethylene bags.

Dissolution profile of the resulting film-coated tablets was recorded using paddle apparatus at 50 rpm and 37°C of the mixed buffer system of the final volume of 500 ml. Type and usage of the buffers are provided in Table 4.

Table 4

| Dissolution stage | Amount of paliperidone released in % |
|---|---|
| 0 to 2 h - acidic buffer 0.1 M HCl | 7 |
| 2 h to 4 h - phosphate buffer of pH 6.8 | 10 |

**Claims**

1. A modified release tablet of paliperidone, **characterised in that** the tablet core comprises:

    a) 0.1% to 6% of paliperidone,
    b) 30% to 40% of a mixture of lightly cross-linked carbomer and highly cross-linked carbomer, wherein the ratio of lightly cross-linked carbomer to highly cross-linked carbomer is from 1:1 to 10:1,
    c) 50 to 65% of filler selected from lactose or dibasic calcium phosphate,
    d) mannitol, talc, silicon dioxide and magnesium stearate.

2. The modified release tablet of paliperidone according to claim 1, wherein the tablet core comprises:

   a) 3% of paliperidone,
   b) 3% of lightly cross-linked powdered carbomer, 3% of highly cross-linked powdered carbomer and 25% of lightly cross-linked granular carbomer,
   c) 59% of lactose
   d) 4% of mannitol, 1% of talc, 1% of silicon dioxide and 1% of magnesium stearate.

3. The modified release tablet of paliperidone according to claim 1 or 2, wherein lightly cross-linked carbomer has viscosity of 4000 to 11000 cP and highly cross-linked carbomer has viscosity of 29400 to 39400 cP when measured as 0.5% aqueous solution neutralized to pH 7.3 - 7.8 with Brookfield viscometer at 20 rpm at 25°C.

4. The modified release tablet of paliperidone according to any preceding claim, wherein the tablet core comprises 6 mg of paliperidone.

5. The modified release tablet of paliperidone according to any preceding claim, wherein the filler is a spray dried lactose monohydrate.

6. The modified release tablet of paliperidone according to any preceding claim, wherein the tablet core releases from 4% to 5% of the total amount of paliperidone per hour, when measured using paddle dissolution apparatus at pH 6.8 in 500 ml of phosphate buffer at 37°C and 50 rpm.

7. The modified release tablet of paliperidone according to any preceding claim, wherein the nominal weight of the tablet core is 200 mg.

8. The modified release tablet of paliperidone according to any preceding claim, wherein the tablet core is further film-coated.

9. The modified release tablet of paliperidone according to claim 8, wherein the film-coating comprises ethylcellulose.

10. The modified release tablet of paliperidone according to claim 8, wherein the film-coating comprises ethylcellulose, a plasticizer and a pore former.

11. The modified release tablet of paliperidone according to claim 10, wherein the pore former is hydroxypropylmethylcellulose.

12. A process for the preparation of a modified release tablet of paliperidone comprising the steps of:

   a) preparation of intragranular phase by: dry blending of paliperidone with a mixture of lightly cross-linked carbomer and highly cross-linked carbomer, mannitol, talc, silicon dioxide, part of filler and part of magnesium stearate; dry compression of the resulting first blend; milling of the tablets obtained to form intragranular phase,
   b) blending of the intragranular phase from step a) with granular lightly cross-linked carbomer, second part of filler and second part of magnesium stearate,
   c) direct tabletting of the second blend obtained in step b)
   d) optionally film-coating of the tablets obtained in step c)
   e) optionally curing the film-coated tablets obtained in step d),

   wherein tablet core obtained in step c) comprises 0.1% to 6% of paliperidone, 30% to 40% of a mixture of lightly cross-linked carbomer and highly cross-linked carbomer, wherein the ratio of lightly cross-linked carbomer to highly cross-linked carbomer is from 1:1 to 10:1 and wherein the tablet core obtained in step c) comprises 50 to 65% of filler selected from lactose or calcium phosphate dibasic.

13. The process according to claim 12, wherein tablet press is used both in step a) and in step c).

Fig. 1

Fig.2

Europäisches
Patentamt

European
Patent Office

Office européen
des brevets

## EUROPEAN SEARCH REPORT

Application Number

EP 17 46 0079

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2011/114213 A1 (INVENTIA HEALTHCARE PRIVATE LTD [IN]; TIWARI SUNIL DEVIPRASAD [IN]; RE) 22 September 2011 (2011-09-22)<br>* examples 1-3 *<br>* claims 1-19 *<br>----- | 1-13 | INV.<br>A61K9/20<br>A61K9/28 |
| Y,D | WO 2010/009900 A1 (KRKA D D NOVO MESTO [SI]; HUDOVORNIK GREGA [SI]; PRESKAR MAJA [SI]; VR) 28 January 2010 (2010-01-28)<br>* example 6A *<br>* claims 1-20 *<br>----- | 1-13 | |
| Y | US 2011/052687 A1 (MEHTA KAMAL [IN] ET AL) 3 March 2011 (2011-03-03)<br>* example 3 *<br>----- | 1-13 | |
| Y | WO 2014/197744 A1 (SYNCHRONEURON INC [US]) 11 December 2014 (2014-12-11)<br>* paragraph [0078] - paragraph [0080] *<br>* claims 1-14 *<br>----- | 1-13 | |
| | | | TECHNICAL FIELDS SEARCHED (IPC)<br><br>A61K |

The present search report has been drawn up for all claims

| Place of search | Date of completion of the search | Examiner |
|---|---|---|
| Munich | 28 May 2018 | Sindel, Ulrike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 17 46 0079

This annex lists the patent family members relating to the patent documents cited in the above-mentioned European search report.
The members are as contained in the European Patent Office EDP file on
The European Patent Office is in no way liable for these particulars which are merely given for the purpose of information.

28-05-2018

| Patent document cited in search report | | Publication date | Patent family member(s) | | Publication date |
|---|---|---|---|---|---|
| WO 2011114213 | A1 | 22-09-2011 | EP | 2547206 A1 | 23-01-2013 |
| | | | US | 2013012524 A1 | 10-01-2013 |
| | | | US | 2016166510 A1 | 16-06-2016 |
| | | | WO | 2011114213 A1 | 22-09-2011 |
| WO 2010009900 | A1 | 28-01-2010 | EP | 2326312 A1 | 01-06-2011 |
| | | | WO | 2010009900 A1 | 28-01-2010 |
| US 2011052687 | A1 | 03-03-2011 | NONE | | |
| WO 2014197744 | A1 | 11-12-2014 | CA | 2914365 A1 | 11-12-2014 |
| | | | CN | 105431144 A | 23-03-2016 |
| | | | EP | 3003297 A1 | 13-04-2016 |
| | | | JP | 2016520653 A | 14-07-2016 |
| | | | US | 2015250746 A1 | 10-09-2015 |
| | | | US | 2016081938 A1 | 24-03-2016 |
| | | | US | 2016101075 A1 | 14-04-2016 |
| | | | WO | 2014197744 A1 | 11-12-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5158952 A
- US 20090048272 A
- WO 199806380 A
- US 20050025831 A
- US 20090202631 A
- EP 2161019 A1
- WO 2010009900 A

**Non-patent literature cited in the description**

- *CHEMICAL ABSTRACTS,* 144598-75-4